(19) Europäisches Patentamt
European Patent Office
Office européen des brevets

(11) EP 4 073 027 B1

(12) # FASCICULE DE BREVET EUROPEEN

(45) Date de publication et mention de la délivrance du brevet:
**30.08.2023 Bulletin 2023/35**

(21) Numéro de dépôt: **20842278.2**

(22) Date de dépôt: **09.12.2020**

(51) Classification Internationale des Brevets (IPC):
***C07C 213/06*** *(2006.01)* ***C07C 219/08*** *(2006.01)*
***C07C 67/03*** *(2006.01)*

(52) Classification Coopérative des Brevets (CPC):
(C-Sets disponibles)
**C07C 213/06** (Cont.)

(86) Numéro de dépôt international:
**PCT/FR2020/052365**

(87) Numéro de publication internationale:
**WO 2021/116608 (17.06.2021 Gazette 2021/24)**

(54) **PROCEDE DE FABRICATION EN CONTINU D'ACRYLATES D'ALKYLE LOURDS**

VERFAHREN ZUR KONTINUIERLICHEN HERSTELLUNG VON SCHWEREN ALKYLACRYLATEN

PROCESS FOR THE CONTINUOUS PREPARATION OF HEAVY ALKYL ACRYLATES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.12.2019 FR 1913924**

(43) Date de publication de la demande:
**19.10.2022 Bulletin 2022/42**

(73) Titulaire: **ARKEMA FRANCE**
**92700 Colombes (FR)**

(72) Inventeurs:
- **BELLINI, Clément**
  **57501 SAINT AVOLD cedex (FR)**
- **MAGET, Sandra**
  **57501 SAINT AVOLD cedex (FR)**
- **DEFER, Patrice**
  **57501 SAINT AVOLD cedex (FR)**

(74) Mandataire: **Arkema Patent**
**Arkema France**
**DRD-DPI**
**420, rue d'Estienne d'Orves**
**92705 Colombes Cedex (FR)**

(56) Documents cités:
**WO-A1-2012/038459** **WO-A1-2017/153653**
**US-A- 3 872 161** **US-A1- 2013 178 592**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Processed by Luminess, 75001 PARIS (FR)

**(Cont. page suivante)**

(52) Classification Coopérative des Brevets (CPC):
(Cont.)

C-Sets
**C07C 213/06, C07C 219/08**

## Description

DOMAINE DE L'INVENTION

La présente invention concerne un procédé de synthèse en continu d'acrylates d'alkyle lourds par réaction de transestérification, à l'aide d'un catalyseur hétérogène.

ARRIERE-PLAN TECHNIQUE

La transestérification est un procédé couramment utilisé pour produire des esters (méth)acryliques. Il est connu de préparer les esters (méth)acryliques de formule (I) :

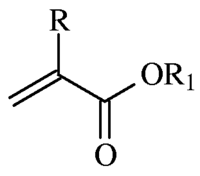

(I)

dans laquelle R est un atome d'hydrogène ou un groupement méthyle, et $R_1$ pouvant être un radical alkyle linéaire ou ramifié, ou un radical aliphatique cyclique, aryle, alkyle-aryle ou aryle-alkyle, pouvant contenir des hétéroatomes, selon un procédé de transestérification par réaction d'un (méth)acrylate d'alkyle de formule (II) :

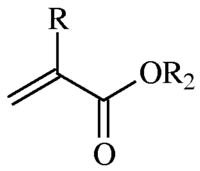

(II)

dans laquelle R a la signification précitée et $R_2$ pouvant être un groupement alkyle linéaire ou ramifié ayant de 1 à 4 atomes de carbone, avec un alcool de formule (III) :

$R_1$-OH (III)

dans laquelle $R_1$ a la signification précitée.

Au cours de la synthèse est généré de l'alcool léger $R_2$-OH, qui est éliminé sous forme d'un azéotrope avec le (méth)acrylate d'alkyle léger (II).

La synthèse des esters (méth)acryliques par transestérification s'effectue généralement en présence d'un catalyseur qui peut être homogène ou hétérogène. Le choix du catalyseur dépend de différents critères, notamment de la nature du (méth)acrylate d'alkyle (II) ou de l'alcool (III) mis en oeuvre, mais aussi de la nature du procédé, tel que procédé en batch ou procédé continu.

Par exemple, les titanates d'alkoxy, décrits dans les documents FR 2777561 et FR 2876375, ou les dérivés d'oxyde d'étain, décrits dans les documents JP 2001172234 et JP 2001187763, permettent de catalyser de façon très sélective la formation d'ester acrylique par transestérification. Dans tous les cas, une étape de purification préliminaire d'élimination du catalyseur par équeutage est nécessaire, car celui-ci est un poison à la purification de l'acrylates d'alkyle lourds par distillation.

Afin de réduire les coûts d'utilisation du catalyseur et de faciliter son recyclage à la réaction, des catalyseurs hétérogènes supportés ont été développés pour la synthèse d'acrylates d'alkyle lourds par réaction de transestérification. Des catalyseurs sur supports polymères métalliques, de titane (FR 2913612) ou de zirconium (EP 0557131) sont utilisés dans un ou plusieurs réacteurs en série de transestérification. Cependant, ces catalyseurs ont des durées de vie assez courtes, du fait des hautes températures associées à la réaction de transestérification.

L'utilisation de sels inorganiques non solubles dans le milieu réactionnel comme catalyseur de transestérification pour la production de (méth)acrylates d'aminoalkyle a été décrite dans le document US 2013178592. L'ajout d'eau à la

réaction (300 - 3000 ppm) permet notamment d'améliorer la sélectivité de ces catalyseurs hétérogènes. Cependant, le $K_3PO_4$ qui est le catalyseur préférentiellement choisi, est très peu sélectif (< 25%) dans la synthèse des esters acryliques.

Le document FR 2175104 (voir aussi US 3872161 A) décrit un procédé de synthèse d'acrylates d'aminoalkyle en présence d'un composé de zinc, dont la teneur molaire est comprise entre 0,01 et 30% par rapport à l'alkylaminoalcool. Parmi les nombreux catalyseurs consistant en composés de zinc listés dans ce document, on trouve le carbonate de zinc basique, décrit dans l'exemple 28. Plus particulièrement, cet exemple décrit la synthèse de diméthylaminoéthylacrylate au moyen d'un procédé de transestérification réalisé en batch, à partir d'acrylate d'éthyle et de diméthylaminoéthanol, en présence de phénothiazine et de carbonate de zinc basique, la teneur de ce dernier étant de 1,045% molaire par rapport au diméthylaminoéthanol. Il est rapporté un rendement de 84,5% en diméthylaminoéthylacrylate. Cependant, l'activité catalytique correspondante, déterminée par rapport à la valeur du TOF (Turnover Frequency), calculée selon la formule indiquée ci-après, est très faible (seulement 9,5 $h^{-1}$).

Par conséquent, il existe à ce jour un besoin de fournir un procédé de transestérification adapté à la fabrication en continu d'acrylates d'alkyle lourds, qui pallie aux inconvénients listés ci-dessus.

A cet effet, l'invention fournit un procédé de synthèse en continu d'acrylates d'alkyle lourds par réaction de transestérification en utilisant l'hydrozincite (de formule $Zn_5(CO_3)_2(OH)_6$ ou $[ZnCO_3]_2 \cdot [Zn(OH)_2]_3$ ou encore $C_2H_{12}O_{12}Zn_5$) comme catalyseur hétérogène. Ce catalyseur permet d'obtenir de très hauts rendements de production lorsqu'il est employé à très faibles teneurs (teneur molaire inférieure à 1% par rapport à l'alcool lourd), tout en gardant une activité catalytique élevée.

De plus, le catalyseur étant séparé du mélange réactionnel par filtration, la purification de l'acrylate d'alkyle désiré se fait en seulement deux étapes (étêtage puis équeutage), au moyen de deux colonnes à distiller, au lieu de trois étapes, tel que rapporté dans l'art antérieur.

## RESUME DE L'INVENTION

L'invention concerne en procédé de synthèse en continu d'esters (méth)acryliques de formule (I) :

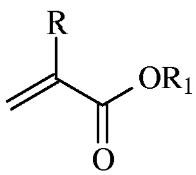

(I)

dans laquelle R est un atome d'hydrogène ou un groupement méthyle, et $R_1$ est un radical alkyle linéaire ou ramifié, ou un radical aliphatique cyclique, aryle, alkyle-aryle ou aryle-alkyle, comportant de 4 à 40 atomes de carbone, ou un radical alkyle linéaire ou ramifié contenant au moins un hétéroatome et de 3 à 40 atomes de carbone, par réaction d'un (méth)acrylate d'alkyle de formule (II) :

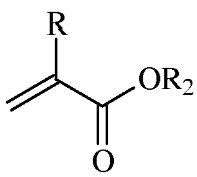

(II)

dans laquelle R a la signification précitée et $R_2$ est un groupement alkyle linéaire ou ramifiée ayant de 1 à 3 atomes de carbone, avec un alcool de formule (III) :

$R_1$-OH (III)

dans laquelle $R_1$ a la signification précitée, en présence d'au moins un inhibiteur de polymérisation et d'hydrozincite comme catalyseur hétérogène.

De manière caractéristique, le procédé met en oeuvre une teneur en catalyseur allant de 0,01 à 0,5% molaire par rapport à l'alcool.

Avantageusement, la purification de l'acrylate d'alkyle se fait en deux étapes : étêtage puis équeutage, au

moyen de deux colonnes à distiller.

La présente invention permet de surmonter les inconvénients de l'état de la technique. Elle fournit plus particulièrement une méthode de synthèse simplifiée, économique et efficace pour fabriquer des acrylates d'alkyles lourds en continu. Elle permet notamment de réduire fortement les coûts d'utilisation du catalyseur, d'améliorer les rendements de production et d'éviter le traitement des résidus métalliques en sortie d'atelier.

De plus, cette méthode, qui nécessite des teneurs très faibles en catalyseurs, présente des rendements en produit final très élevés. L'efficacité catalytique, quantifiée au moyen du TOF, est fortement améliorée, et s'élève à des valeurs supérieures à 50 $h^{-1}$.

## DESCRIPTION DE MODES DE REALISATION DE L'INVENTION

L'invention est maintenant décrite plus en détail et de façon non limitative dans la description qui suit.

La synthèse d'acrylates d'alkyle lourds par réaction de transestérification par catalyse homogène implique une étape préalable d'élimination du catalyseur par distillation (équeutage). Une partie du pied de cette distillation contenant le résidu métallique est envoyé en destruction (hors recyclage), augmentant nettement les coûts d'utilisation du catalyseur et l'impact écologique associé.

D'autre part, la tête de cette distillation contient l'acrylate d'alkyle lourd désiré, ainsi que l'acrylate d'alkyle léger et l'alcool lourd n'ayant pas réagi. Une distillation supplémentaire (étêtage) est nécessaire afin de séparer l'acrylate d'alkyle lourd des matières premières plus légères. Le pied de cette seconde distillation contient alors l'acrylate d'alkyle lourd désiré et le/les l'inhibiteur(s) de polymérisation en excès. Une dernière étape de distillation (purification) est alors nécessaire afin d'obtenir l'acrylate d'alkyle lourd désiré dans les spécifications souhaitées.

L'élimination du catalyseur directement à la réaction permettrait donc de réaliser ce procédé de purification en seulement deux étapes (étêtage puis équeutage) au lieu de trois, abaissant le coût de construction d'un atelier de production, ainsi que les coûts liés à l'énergie nécessaire à la production.

La présente invention concerne un procédé de synthèse en continu d'acrylates d'alkyle lourds par réaction de transestérification en utilisant l'hydrozincite comme catalyseur hétérogène, la purification du produit final étant réalisée en deux étapes.

L'invention concerne un procédé de synthèse en continu d'esters (méth)acryliques de formule (I) :

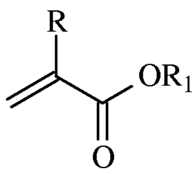

(I)

dans laquelle R est un atome d'hydrogène ou un groupement méthyle, et $R_1$ est un radical alkyle linéaire ou ramifié, ou un radical aliphatique cyclique, aryle, alkyle-aryle ou aryle-alkyle, comportant de 4 à 40 atomes de carbone, ou un radical alkyle linéaire ou ramifié contenant au moins un hétéroatome et de 3 à 40 atomes de carbone, par réaction d'un (méth)acrylate d'alkyle de formule (II) :

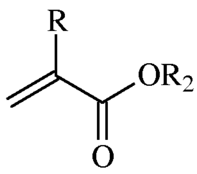

(II)

dans laquelle R a la signification précitée et $R_2$ est un groupement alkyle linéaire ou ramifiée ayant de 1 à 3 atomes de carbone, avec un alcool de formule (III) :

$R_1$-OH (III)

en présence d'au moins un inhibiteur de polymérisation et d'hydrozincite comme catalyseur hétérogène, caractérisé en ce que la teneur en catalyseur est comprise entre 0,01 et 0,5% molaire par rapport à l'alcool.

Selon diverses réalisations, ledit procédé comprend les caractères suivants, le cas échéant combinés.

Selon un mode de réalisation, la réaction de transestérification a lieu dans un réacteur agité à une température de 80-150°C, de préférence entre 90 et 130°C, le mélange réactionnel en sortie du réacteur comprenant l'acrylate d'alkyle lourd avec, comme produits légers, l'alcool lourd, l'alcool léger et l'acrylate léger n'ayant pas réagi, et, comme produits lourds, le ou les inhibiteurs de polymérisation ainsi que des produits de réaction lourds. Le mélange azéotropique acrylate léger / alcool léger est éliminé par une colonne de distillation (colonne azéotropique) surmontant le réacteur. Le mélange réactionnel est soumis à une étape de séparation liquide/solide pour séparer le catalyseur.

Le catalyseur peut être introduit à la réaction en suspension au mélange réactionnel ou alors placé en lit fixe dans le ou les réacteurs de transestérification. Les techniques usuelles de séparation liquide/solide (filtration, électrofiltration, absorption, centrifugation ou décantation) permettent d'extraire un produit brut en sorti de réacteur exempt ou sensiblement exempt de catalyseur (teneur en catalyseur inférieure à 500 ppm).

Selon un mode de réalisation, l'inhibiteur de polymérisation comprend au moins un composé N-oxyl et au moins un inhibiteur de polymérisation autre qu'un composé N-oxyl.

Le composé N-oxyl est choisi parmi le 2,2,6,6-tétraméthylpipéridine 1-oxyl (dénommé TEMPO), et ses dérivés tels que le 4-Hydroxy-2,2,6,6-tétraméthylpipéridine 1-oxyl (4-OH-TEMPO ou 4-HT), ou le 4-oxo-2,2,6,6-tétraméthylpipéridine 1-oxyl (4-Oxo-TEMPO), ainsi qu'un mélange de ces composés.

Selon un mode de réalisation, l'inhibiteur de polymérisation est choisi parmi les composés phénoliques et les composés phénothiazine.

Par composé phénolique on entend les composés dérivés du phénol, du naphtol et les quinones. Comme composés phénoliques, on peut citer les composés suivants sans que cette liste soit limitative : p-aminophénol, p-nitrosophénol, 2-tert-butylphénol, 4-tert-butylphénol, 2,4-di-tert-butylphénol, 2-méthyl-4-tert-butyéphénol, 4-méthyl-2,6-tert-butylphénol (ou 2,6-tert-butyl-p-crésol) or 4-tert-butyl-2,6-diméthylphénol, hydroquinone (HQ), éther méthylique d'hydroquinone (EMHQ).

Par composé phénothiazine, on entend la phénothiazine et ses dérivés, de préférence la phénothiazine (PTZ). De préférence, la composition stabilisante comprend au moins un inhibiteur de polymérisation choisi parmi la phénothiazine, le 4-méthyl-2,6-tert-butylphénol, l'hydroquinone et l'éther méthylique d'hydroquinone.

Selon un mode de réalisation, le composé N-oxyl est en excès dans la composition stabilisante.

Selon un mode de réalisation, le rapport massique entre le composé N-oxyl et l'(ou les) inhibiteur(s) de polymérisation est compris entre 1 et 10, de préférence entre 2 et 10, plus préférentiellement entre 4 et 10, en particulier entre 5 et 10, le rapport massique étant exprimé bornes comprises.

Avantageusement, la teneur massique totale en inhibiteur de polymérisation est de 0,001 à 0,5% dans le mélange réactionnel.

Avantageusement, l'ester (méth)acrylique de formule (I) est purifié suivant les étapes ci-dessous:

a. on adresse à une première colonne de distillation (C1) sous pression réduite (de 1000 à 20000 Pa) ledit mélange réactionnel, et on y effectue une distillation permettant d'obtenir:

- o en tête, un flux comprenant des matières premières n'ayant pas réagi, et une fraction minoritaire de produits lourds (les adduits de Michael), et
- o en pied, un flux de composé essentiellement de l'acrylate d'alkyle lourd ;

b. on adresse le flux de pied de la première colonne de distillation (C1) à une seconde colonne de distillation (C2) sous pression réduite (de 1000 à 20000 Pa), dans laquelle est effectuée une distillation permettant d'obtenir :

- ◦ en tête, l'acrylate d'alkyle lourd désiré,
- ◦ en pied, l'inhibiteur de polymérisation, ainsi que des produits de réaction lourds.

Selon un mode de réalisation, le procédé selon l'invention comprend une étape additionnelle dans laquelle le flux de tête de la première colonne de distillation (C1) est envoyé au réacteur de transestérification afin de recycler les matières premières.

Selon un mode de réalisation, dans le réacteur le rapport molaire entre le (méth)acrylate d'alkyle de formule (II) (appelé « acrylate d'alkyl lourd ») et l'alcool de formule (III) (appelé « alcool lourd ») est compris entre 1,1 et 3, de préférence entre 1,7 et 2,2.

Selon un mode de réalisation, l'ester (méth)acrylique de formule (I) est l'acrylate de diméthylaminoéthyle.

Selon un mode de réalisation, l'alcool est le diméthylaminoéthanol.

De manière surprenante, il a été constaté que le procédé selon l'invention permet de diminuer fortement la formation d'adduits de Michael, qui résultent des réactions d'addition de Michael d'une molécule d'alcool (contenant un atome d'hydrogène labile) sur la double liaison de l'ester (méth)acrylique, lors de la réaction de transestérification de

dérivés (méth)acryliques. Ceci est observé notamment par rapport à la formation d'adduits de Michael en présence de fortes concentration de catalyseur carbonate de zinc basique.

Par exemple, dans le cas de la fabrication de l'ADAME par transestérification entre un acrylate léger, tel que l'acrylate de méthyle (AM) ou l'acrylate d'éthyle (AE), et le N, N-diméthyl aminoéthanol (DMAE), l'alcool n'ayant pas encore réagi ou les alcools légers générés au cours de la réaction (méthanol ou éthanol) s'ajoutent à la double liaison de l'ADAME déjà formé ou de l'acrylate léger (AM ou AE) n'ayant pas réagi, pour former des sous-produits lourds d'addition de Michael [DMAE+ADAME].

Ces sous-produits lourds sont généralement concentrés dans une « fraction lourde », séparée au cours du processus de purification de l'ADAME brut. L'élimination de cette fraction lourde pose généralement un problème car elle nécessite d'être incinérée, et entraine des pertes importantes de matières premières (notamment DMAE) et de produit fini (ADAME) qui sont présents dans cette fraction sous forme libre ou sous forme d'adduits de Michael.

Or, les mesures de la concentration en adduits de ce type dans le flux de pied de la première colonne de distillation (C1) du procédé selon l'invention montrent que leur teneur massique ne dépasse pas 5%, valeur qui est significativement plus faible que celles mesurées dans les fractions lourdes obtenues par le procédé décrit dans le document FR 2175104, et lorsque la teneur du catalyseur est supérieure à 0,5% molaire par rapport à l'alcool.

La teneur en adduits de Michael est mesurée par chromatographie en phase gazeuse. Par ailleurs, il a été constaté que le procédé selon l'invention mettant en oeuvre l'hydrozincite comme catalyseur hétérogène à très faibles teneurs (teneur molaire allant de 0,01 à 0,5%, bornes comprises, par rapport à l'alcool lourd), permet d'obtenir de très hauts rendements de production, ceci s'accompagnant d'une activité catalytique élevée. Plus précisément, l'activité catalytique est déterminée par la valeur do TOF (Turn Over Frequency), calculé selon la formule ci-dessous. Le temps de séjour est déterminé en fonction du débit d'extraction en pied du réacteur de transestérification.

$$\boldsymbol{TOF = \frac{N_{ADAME}}{N_{Catalyseur} \times TdS}}$$

Le procédé selon l'invention se caractérise par des valeurs du TOF supérieures à $50h^{-1}$.

<u>EXEMPLES</u>

Les exemples suivants illustrent l'invention sans la limiter.

<u>Protocole général pour les exemples 1 à 3</u>

Dans les exemples suivants, les abréviations suivantes ont été utilisées :

AE : Acrylate d'Ethyle
DMAE : Diméthylaminoéthanol
ADAME : Acrylate de diméthylaminoéthyle
PTZ : Phénothiazine
4-HT : 4-OH-TEMPO
$Zn_5(CO_3)_2(OH)_6$ : Hydrozincite
$Ti(OET)_4$ : Tétraéthyle titanate
TOF : Turn Over Frequency (efficacité ou activité catalytique)

Dans un réacteur agité de 0,5 L, chauffé par circulation d'huile thermostatée à 135°C dans une double enveloppe, surmonté d'une colonne à distiller à garnissage Multiknit, avec condenseur à eau glycolée en tête de colonne, tête de reflux, séparateur à vide, recettes et pièges, on introduit en continu l'AE, l'alcool lourd (DMAE) et un catalyseur de transestérification ($Ti(OET)_4$ ou $Zn_5(CO_3)_2(OH)_6$). Les inhibiteurs de polymérisation (PTZ, 4-HT, EMHQ ou un mélange de ces composés) sont injectés en continu en tête en mélange avec l'AE.

Durant toute la synthèse, on effectue un bullage d'air dans le mélange réactionnel. La réaction est effectuée à une température de 119-121°C sous un vide de 86 à 87 kPa (860 à 870 mbar). L'éthanol formé au cours de la réaction est éliminé au fur et à mesure de sa formation sous forme d'un azéotrope AE/éthanol. Le taux de conversion est suivi par analyse réfractométrique de l'azéotrope. La teneur en éthanol est comprise entre 58% et 62%. Le brut réactionnel est extrait à l'aide d'un débordement et récupéré dans une recette.

La teneur en alcool lourd, (méth)acrylate lourds (ADAME) et éthanol des flux de distillat et de pied sont analysés après 120 h de marche par Chromatographie en phase gaz afin de déterminer le rendement en ester désiré, la conversion de l'alcool lourds et la sélectivité. Les inhibiteurs sont analysés et quantifiés par méthode HPLC. La quantification des

impuretés lourdes (catalyseur + polymère) est réalisée à l'aide d'une balance thermique. Toutes les concentrations sont données en pourcentages massique et ppm massique sauf mention contraire.

A partir de ces analyses, l'activité catalytique au bout de 120 h est également déterminée par la valeur du TOF, calculée au moyen de la formule indiquée ci-dessus.

Exemple 1 - Synthèse d'ADAME en continu avec hydrozincite à 0,2 mol-% (selon invention)

Le catalyseur est introduit dans le bac d'alimentation en suspension et agité mécaniquement. Le mélange réactionnel contenant l'AE (61,7 g/h), la DMAE (34,2 g/h) et la catalyseur (0,4 g/h) sont alors introduits en continu dans le réacteur.

La température est maintenue dans le réacteur à 120 °C durant 120 h et le mélange réactionnel est récupéré par débordement (78,4 g/h) dans un bac de stockage. A la fin des 120 h, le mélange réactionnel, ainsi que le mélange azéotropique sont analysés, permettant ainsi d'obtenir les résultats suivants :

- Rendement ADAME = 82,0%
- Conversion DMAE = 80,8%
- Sommes des adduits = 4,5%
- TOF = 71,6 $h^{-1}$.

Exemple 2 - Synthèse d'ADAME en continu avec hydrozincite à 0,8 mol-% (comparatif)

Le catalyseur est introduit dans le bac d'alimentation en suspension et agité mécaniquement. Le mélange réactionnel contenant l'AE (64,3 g/h), la DMAE (35,7 g/h) et la catalyseur (1,7 g/h) sont alors introduits en continu dans le réacteur.

La température est maintenue dans le réacteur à 120 °C durant 120 h et le mélange réactionnel est récupéré par débordement (78,5 g/h) dans un bac de stockage. A la fin des 120 h, le mélange réactionnel, ainsi que le mélange azéotropique sont analysés, permettant ainsi d'obtenir les résultats suivants :

- Rendement ADAME = 83,4%
- Conversion DMAE = 83,7%
- Sommes des adduits = 7,7%
- TOF = 18,3 $h^{-1}$.

Exemple 3 - Synthèse d'ADAME en continu avec $Ti(OET)_4$ à 0,2 mol-% (comparatif)

Le catalyseur $Ti(OET)_4$ est introduit dans le bac d'alimentation de façon homogène. Le mélange réactionnel contenant l'AE (64,2 g/h), la DMAE (35,6 g/h) et la catalyseur (0,2 g/h) sont alors introduits en continu dans le réacteur.

La température est maintenue dans le réacteur à 120 °C durant 120 h et le mélange réactionnel est récupéré par débordement (82,5 g/h) dans un bac de stockage. A la fin des 120 h, le mélange réactionnel, ainsi que le mélange azéotropique sont analysés, permettant ainsi d'obtenir les résultats suivants :

- Rendement ADAME = 53,6%
- Conversion DMAE = 55,5%
- Somme des adduits = 0,7%
- TOF = 45,5 $h^{-1}$.

L'activité catalytique est plus élevée (TOF supérieurs) lorsque la concentration en hydrozincite est abaissée à 0,2 mol-% par rapport à la DMAE (exemple 1), ce qui n'est pas le cas pour le titanate d'éthyle (exemple 3), du fait de la perte de rendement.

De plus, la formation d'adduits de Michael est plus faible lorsque la concentration en hydrozincite est abaissée à 0,2 mol-% par rapport à la DMAE (exemple 1) en comparaison à une concentration plus élevée (exemple comparatif 2). Cette correspondance entre concentration en catalyseur et formation d'adduits de Michael n'est pas observée avec le titanate d'éthyle (exemple comparatif 3).

Exemples 4 et 5

Exemple 4 - Purification du brut réactionnel $Zn_5(CO_3)_2(OH)_6$ après filtration (selon invention)

Un échantillon du mélange brut réactionnel (0,5 L) obtenu après 120 h dans l'exemple 2 est filtré pour enlever le catalyseur en suspension. Le filtrat est ensuite placé dans un réacteur agité de 0,5 L, chauffé par circulation d'huile thermostatée à 100 °C dans une double enveloppe, surmonté d'une colonne à distiller à garnissage Multiknit, avec condenseur à eau glycolée en tête de colonne, tête de reflux, séparateur à vide, recettes et pièges.

Le mélange réactionnel est ensuite distillé sous un vide poussé (32 à 15 kPa) afin d'extraire une première fraction en tête de colonne composée majoritairement d'AE. La même opération est effectuée sous un vide plus poussé (15 à 4 kPa) fin d'extraire une seconde fraction en tête de colonne composée majoritairement de DMAE.

Enfin, l'ADAME est purifié par distillation en appliquant un vide à 12 kPa en montant la température de l'huile à 140°C. L'acrylate purifié est récolté dans une recette en tête de colonne. L'analyse GC donne la composition suivante :

- Pureté ADAME = 99,9%
- AE résiduel = 243 ppm
- DMAE résiduel = 557 ppm.

La pureté de l'ADAME est acceptable (> 99,8%), et la teneur des produits légers AE/DMAE issus de la décomposition de l'ADAME par action du catalyseur est sous les spécifications recherchées pour ce produit (300 ppm pour l'AE et 1000 ppm pour la DMAE).

Exemple 5 - Purification du brut réactionnel $Ti(OET)_4$ (contre-exemple)

Un échantillon du mélange brut réactionnel (0,5 L) obtenu après 120 h dans l'exemple 3 est placé dans un réacteur agité de 0,5 L, chauffé par circulation d'huile thermostatée à 100 °C dans une double enveloppe, surmonté d'une colonne à distiller à garnissage Multiknit, avec condenseur à eau glycolée en tête de colonne, tête de reflux, séparateur à vide, recettes et pièges.

L'étape de filtration n'est pas effectuée car le catalyseur $Ti(OET)_4$ est soluble à froid.

L'analyse GC de l'ADAME donne la composition suivante :

- Pureté ADAME = 99,7%
- AE résiduel = 1602 ppm
- DMAE résiduel = 1016 ppm

La pureté de l'ADAME n'est pas acceptable (< 99,8%), et la teneur des produits légers AE/DMAE issus de la décomposition de l'ADAME par action du catalyseur est supérieure aux spécifications recherchées pour ce produit (300 ppm pour l'AE et 1000 ppm pour la DMAE).

## Revendications

**1.** Procédé de synthèse en continu d'esters (méth)acryliques de formule (I) :

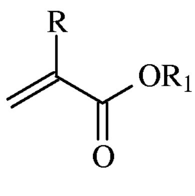

(I)

dans laquelle R est un atome d'hydrogène ou un groupement méthyle, et $R_1$ est un radical alkyle linéaire ou ramifié, ou un radical aliphatique cyclique, aryle, alkyle-aryle ou aryle-alkyle, comportant de 4 à 40 atomes de carbone, ou un radical alkyle linéaire ou ramifié contenant au moins un hétéroatome et de 3 à 40 atomes de carbone, par réaction d'un (méth)acrylate d'alkyle de formule (II) :

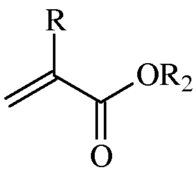

(II)

dans laquelle R a la signification précitée et $R_2$ est un groupement alkyle linéaire ou ramifiée ayant de 1 à 3 atomes de carbone, avec un alcool de formule (III) :

$R_1$-OH (III)

en présence d'au moins un inhibiteur de polymérisation et d'hydrozincite comme catalyseur hétérogène, **caractérisé en ce que** la teneur en catalyseur est comprise entre 0,01 et 0,5% molaire par rapport à l'alcool.

**2.** Procédé selon la revendication 1, dans lequel la réaction de transestérification a lieu dans un réacteur agité à une température de 80-150°C, le mélange réactionnel en sortie du réacteur comprenant l'acrylate d'alkyle lourd avec, comme produits légers, l'alcool lourd, l'alcool léger et l'acrylate léger n'ayant pas réagi, et, comme produits lourds, le ou les inhibiteurs de polymérisation ainsi que des produits de réaction lourds, ledit mélange réactionnel étant soumis à une étape de séparation liquide/solide pour séparer le catalyseur.

**3.** Procédé selon la revendication 2, dans lequel ledit ester (méth)acrylique de formule (I) est purifié suivant les étapes ci-dessous:

a. on adresse à une première colonne de distillation (C1) sous pression de 1000 à 20000 Pa ledit mélange réactionnel, et on y effectue une distillation permettant d'obtenir:

- en tête, un flux comprenant des matières premières n'ayant pas réagi, et une fraction minoritaire de produits lourds (les adduits de Michael), et
- en pied, un flux de composé essentiellement de l'acrylate d'alkyle lourd ;

b. on adresse le flux de pied de la première colonne de distillation (C1) à une seconde colonne de distillation (C2) sous pression de 1000 à 20000 Pa, dans laquelle est effectuée une distillation permettant d'obtenir :

- en tête, l'acrylate d'alkyle lourd désiré,
- en pied, l'inhibiteur de polymérisation, ainsi que des produits de réaction lourds.

**4.** Procédé selon la revendication 3, comprenant une étape additionnelle dans laquelle le flux de tête de la première colonne de distillation (C1) est envoyé au réacteur de transestérification afin de recycler les matières premières.

**5.** Procédé selon la revendication 2, dans lequel ladite étape de séparation liquide/solide met en oeuvre une technique choisie dans la liste: filtration, électrofiltration, absorption, centrifugation et décantation, la teneur en catalyseur du produit brut ainsi traité étant inférieure à 500 ppm.

**6.** Procédé selon l'une des revendications 2 à 5, dans lequel ledit catalyseur est introduit à la réaction en suspension au mélange réactionnel ou il est placé en lit fixe dans le réacteur de transestérification.

**7.** Procédé selon l'une des revendications 1 à 6, dans lequel ledit inhibiteur de polymérisation utilisé comprend au moins un dérivé N-oxyl et au moins un inhibiteur de polymérisation choisi parmi les composés phénoliques et les composés phénothiazine, dans un rapport massique compris entre 1 et 10, bornes comprises.

**8.** Procédé selon la revendication 7, dans lequel le composé N-oxyl est choisi parmi le 2,2,6,6-tétraméthylpipéridine 1-oxyl (dénommé TEMPO), et ses dérivés tels que le 4-Hydroxy-2,2,6,6-tétraméthylpipéridine 1-oxyl (4-OH-TEMPO) ou le 4-oxo-2,2,6,6-tétraméthylpipéridine 1-oxyl (4-Oxo-TEMPO), ainsi qu'un mélange de ces composés.

**9.** Procédé selon l'une des revendications 1 à 6, dans lequel ledit inhibiteur de polymérisation utilisé est choisi parmi les composés phénoliques et les composés phénothiazine.

**10.** Procédé selon l'une quelconque des revendications précédentes dans lequel l'inhibiteur de polymérisation est choisi parmi la phénothiazine, le 4-méthyl-2,6-tert-butylphénol, l'hydroquinone, l'éther méthylique d'hydroquinone, p-aminophénol, p-nitrosophénol, 2-tert-butylphénol, 4-tert-butylphénol, 2,4-di-tert-butylphénol, 2-méthyl-4-tert-butyéphénol, 4-méthyl-2,6-tert-butylphénol (ou 2,6-tert-butyl-p-crésol) or 4-tert-butyl-2,6-diméthylphénol, hydroquinone (HQ), et l'éther méthylique d'hydroquinone (EMHQ).

**11.** Procédé selon l'une des revendications 1 à 7, dans lequel la teneur massique en inhibiteur de polymérisation est de 0,001 à 0,5% dans le mélange réactionnel.

**12.** Procédé selon l'une des revendications précédentes, dans lequel la teneur massique en adduits de Michael dans le flux de pied de la première colonne de distillation (C1) est inférieure ou égale à 5%.

**13.** Procédé selon l'une des revendications précédentes, dans lequel l'ester (méth)acrylique de formule (I) est l'acrylate de diméthylaminoéthyle.

**Patentansprüche**

**1.** Verfahren zur kontinuierlichen Synthese von (Meth)-acrylsäureestern der Formel (I):

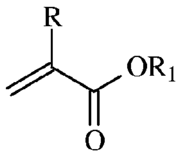

(I)

in der R für ein Wasserstoffatom oder eine Methylgruppe steht und $R_1$ für einen linearen oder verzweigten Alkylrest oder einen cycloaliphatischen Rest, Arylrest, Alkylarylrest oder Arylalkylrest mit 4 bis 40 Kohlenstoffatomen oder einen linearen oder verzweigten Alkylrest mit mindestens einem Heteroatom und 3 bis 40 Kohlenstoffatomen steht, durch Umsetzung eines Alkyl(meth)acrylats der Formel (II):

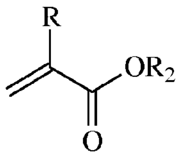

(II)

in der R die oben angegebene Bedeutung hat und $R_2$ für eine lineare oder verzweigte Alkylgruppe mit 1 bis 3 Kohlenstoffatomen steht, mit einem Alkohol der Formel (III):

$R_1$-OH (III)

in Gegenwart von mindestens einem Polymerisationsinhibitor und Hydrozincit als heterogenem Katalysator, **dadurch gekennzeichnet, dass** der Katalysatorgehalt zwischen 0,01 und 0,5 Mol-% bezogen auf den Alkohol, liegt.

**2.** Verfahren nach Anspruch 1, wobei die Umesterungsreaktion in einem Rührreaktor bei einer Temperatur von 80-150°C durchgeführt wird, wobei die Reaktionsmischung am Ausgang des Reaktors das schwere Alkylacrylat mit dem schweren Alkohol, dem leichten Alkohol und dem nicht umgesetzten leichten Acrylat als leichte Produkte und dem Polymerisationsinhibitor bzw. den Polymerisationsinhibitoren sowie schweren Reaktionsprodukten als schwere Produkte umfasst, wobei die Reaktionsmischung einem Flüssig/Fest-Trennungsschritt zur Abtrennung des Katalysators unterworfen wird.

**3.** Verfahren nach Anspruch 2, wobei der (Meth)acrylsäureester der Formel (I) gemäß den nachstehenden Schritten gereinigt wird:

a. man führt die Reaktionsmischung einer ersten Destillationssäule (C1) unter einem Druck von 1000 bis 20.000 Pa zu und führt darin eine Destillation durch, wobei Folgendes erhalten wird:

- am Kopf ein Strom, der nicht umgesetzte Ausgangsstoffe und eine kleinere Fraktion von schweren Produkten (den Michael-Addukten) umfasst, und
- am Sumpf ein Strom, der im Wesentlichen aus dem schweren Alkylacrylat besteht;

b. man führt den Sumpfstrom aus der ersten Destillationssäule (C1) einer zweiten Destillationssäule (C2) unter einem Druck von 1000 bis 20.000 Pa zu, in der eine Destillation durchgeführt wird, wobei Folgendes erhalten wird:

- am Kopf das gewünschte schwere Alkylacrylat und
- am Sumpf der Polymerisationsinhibitor sowie schwere Reaktionsprodukte.

**4.** Verfahren nach Anspruch 3, umfassend einen zusätzlichen Schritt, wobei der Kopfstrom aus der ersten Destillationssäule (C1) zur Rezyklierung der Ausgangsstoffe dem Umesterungsreaktor zugeführt wird.

**5.** Verfahren nach Anspruch 2, wobei bei dem Flüssig/Fest-Trennschritt eine aus der folgenden Liste ausgewählte Technik verwendet wird: Filtration, Elektrofiltration, Absorption, Zentrifugation und Dekantierung, wobei der Katalysatorgehalt des so behandelten Rohprodukts weniger als 500 ppm beträgt.

**6.** Verfahren nach einem der Ansprüche 2 bis 5, wobei der Katalysator als Suspension in der Reaktionsmischung in den Ansatz eingetragen oder in einem Festbett in dem Umesterungsreaktor angeordnet wird.

**7.** Verfahren nach einem der Ansprüche 1 bis 6, wobei der verwendete Polymerisationsinhibitor mindestens ein N-Oxyl-Derivat und mindestens einen aus Phenolverbindungen und Phenothiazinverbindungen ausgewählten Polymerisationsinhibitor in einem Massenverhältnis zwischen 1 und 10 einschließlich der Grenzen umfasst.

**8.** Verfahren nach Anspruch 7, wobei die N-Oxylverbindung aus 2,2,6,6-Tetramethylpiperidin-1-oxyl (als TEMPO bezeichnet) und Derivaten davon wie 4-Hydroxy-2,2,6,6-tetramethylpiperidin-1-oxyl (4-OH-TEMPO) oder 4-Oxo-2,2,6,6-tetramethylpiperidin-1-oxyl (4-Oxo-TEMPO) sowie einer Mischung dieser Verbindungen ausgewählt wird.

**9.** Verfahren nach einem der Ansprüche 1 bis 6, wobei der verwendete Polymerisationsinhibitor aus Phenolverbindungen und Phenothiazinverbindungen ausgewählt wird.

**10.** Verfahren nach einem der vorhergehenden Ansprüche, wobei der Polymerisationsinhibitor aus Phenothiazin, 4-Methyl-2,6-tert-butylphenol, Hydrochinon, Hydrochinonmethylether, p-Aminophenol, p-Nitrosophenol, 2-tert-Butylphenol, 4-tert-Butylphenol, 2,4-Di-tert-butylphenol, 2-Methyl-4-tert-butylphenol, 4-Methyl-2,6-tert-butylphenol (oder 2,6-tert-Butyl-p-kresol) oder 4-tert-Butyl-2,6-dimethylphenol, Hydrochinon (HQ) und Hydrochinonmethylether (HQME) ausgewählt ist.

**11.** Verfahren nach einem der Ansprüche 1 bis 7, wobei der Massengehalt an Polymerisationsinhibitor in der Reaktionsmischung 0,001 bis 0,5 % beträgt.

**12.** Verfahren nach einem der vorhergehenden Ansprüche, wobei der Massengehalt an Michael-Addukten in dem Sumpfstrom aus der ersten Destillationssäule (C1) kleiner oder gleich 5 % ist.

**13.** Verfahren nach einem der vorhergehenden Ansprüche, wobei es sich bei dem (Meth)acrylsäureester der Formel (I) um Dimethylaminoethylacrylat handelt.

## Claims

**1.** A process for the continuous synthesis of (meth)acrylic esters of formula (I):

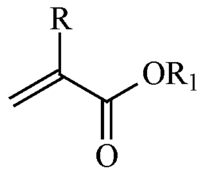

(I)

in which R is a hydrogen atom or a methyl group, and $R_1$ is a linear or branched alkyl radical, or a cycloaliphatic, aryl, alkylaryl or arylalkyl radical, including from 4 to 40 carbon atoms, or a linear or branched alkyl radical containing at least one heteroatom and from 3 to 40 carbon atoms, by reaction of an alkyl (meth)acrylate of formula (II):

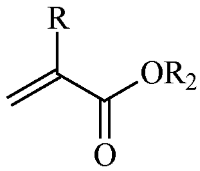

(II)

in which R has the abovementioned meaning and $R_2$ is a linear or branched alkyl group containing from 1 to 3 carbon atoms, with an alcohol of formula (III):

$R_1$-OH (III)

in the presence of at least one polymerization inhibitor and of hydrozincite as heterogeneous catalyst, **characterized in that** the catalyst content is between 0.01 mol% and 0.5 mol% relative to the alcohol.

**2.** The process as claimed in claim 1, in which the transesterification reaction takes place in a reactor stirred at a temperature of 80-150°C, the reaction mixture exiting the reactor comprising the heavy alkyl acrylate with, as light products, the heavy alcohol, the light alcohol and the unreacted light acrylate, and, as heavy products, the polymerization inhibitor(s) and also the heavy reaction products, said reaction mixture being subjected to a liquid/solid separation step to separate out the catalyst.

**3.** The process as claimed in claim 2, in which said (meth)acrylic ester of formula (I) is purified according to the steps below:

a. said reaction mixture is sent to a first distillation column (C1) under a pressure of 1000 to 20 000 Pa, and a distillation is performed therein, producing:

- at the top, a stream comprising unreacted starting materials, and a minor fraction of heavy products (the Michael adducts), and
- at the tail, a stream composed essentially of the heavy alkyl acrylate;

b. the tail stream from the first distillation column (C1) is sent to a second distillation column (C2) under a pressure of 1000 to 20 000 Pa, in which a distillation is performed producing:

- at the top, the desired heavy alkyl acrylate,
- at the tail, the polymerization inhibitor, and also heavy reaction products.

**4.** The process as claimed in claim 3, comprising an additional step in which the head stream from the first distillation column (C1) is sent to the transesterification reactor in order to recycle the starting materials.

**5.** The process as claimed in claim 2, in which said liquid/solid separation step involves a technique chosen from the following list: filtration, electrofiltration, absorption, centrifugation and decantation, the catalyst content of the crude product thus treated being less than 500 ppm.

6. The process as claimed in one of claims 2 to 5, in which said catalyst is introduced into the reaction as a suspension in the reaction mixture or it is placed in a fixed bed in the transesterification reactor.

7. The process as claimed in one of claims 1 to 6, in which said polymerization inhibitor used comprises at least one N-oxyl derivative and at least one polymerization inhibitor chosen from phenol compounds and phenothiazine compounds in a mass ratio of between 1 and 10, limits included.

8. The process as claimed in claim 7, in which the N-oxyl compound is chosen from 2,2,6,6-tetramethylpiperidine 1-oxyl (called TEMPO), and derivatives thereof such as 4-hydroxy-2,2,6,6-tetramethylpiperidine 1-oxyl (4-OH-TEMPO) or 4-oxo-2,2,6,6-tetramethylpiperidine 1-oxyl (4-Oxo-TEMPO), and also a mixture of these compounds.

9. The process as claimed in one of claims 1 to 6, in which said polymerization inhibitor used is chosen from phenol compounds and phenothiazine compounds.

10. The process as claimed in any one of the preceding claims, in which the polymerization inhibitor is chosen from phenothiazine, 4-methyl-2,6-tert-butylphenol, hydroquinone, hydroquinone methyl ether, p-aminophenol, p-nitrosophenol, 2-tert-butylphenol, 4-tert-butylphenol, 2,4-di-tert-butylphenol, 2-methyl-4-tert-butylphenol, 4-methyl-2,6-tert-butylphenol (or 2,6-tert-butyl-p-cresol) or 4-tert-butyl-2,6-dimethylphenol, hydroquinone (HQ), and hydroquinone methyl ether (HQME).

11. The process as claimed in one of claims 1 to 7, in which the mass content of polymerization inhibitor is from 0.001% to 0.5% in the reaction mixture.

12. The process as claimed in one of the preceding claims, in which the mass content of Michael adducts in the tail stream from the first distillation column (C1) is less than or equal to 5%.

13. The process as claimed in one of the preceding claims, in which the (meth)acrylic ester of formula (I) is dimethylaminoethyl acrylate.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- FR 2777561
- FR 2876375
- JP 2001172234 B
- JP 2001187763 B
- FR 2913612
- EP 0557131 A
- US 2013178592 A
- FR 2175104
- US 3872161 A